(19) **Europäisches Patentamt / European Patent Office / Office européen des brevets**

(11) **EP 3 706 618 B1**

(12) **EUROPEAN PATENT SPECIFICATION**

(45) Date of publication and mention of the grant of the patent:
**24.12.2025 Bulletin 2025/52**

(21) Application number: **18897763.1**

(22) Date of filing: **21.12.2018**

(51) International Patent Classification (IPC):
**A61B 5/00** *(2006.01)*　　**A61B 5/024** *(2006.01)*

(52) Cooperative Patent Classification (CPC):
**A61B 5/721; A61B 5/02416; A61B 5/0816; A61B 5/6803; A61B 5/681; A61B 5/6815; A61B 5/6824; A61B 5/6829; A61B 5/6898; A61B 5/7221; A61B 5/7278;** A61B 5/02405; A61B 5/11; A61B 5/352; A61B 5/725;　　(Cont.)

(86) International application number:
**PCT/US2018/067127**

(87) International publication number:
**WO 2019/133491 (04.07.2019 Gazette 2019/27)**

(54) **METHODS OF DETERMINING PHYSIOLOGICAL INFORMATION BASED ON BAYESIAN PEAK SELECTION AND MONITORING DEVICES INCORPORATING THE SAME**

VERFAHREN ZUR BESTIMMUNG VON PHYSIOLOGISCHEN INFORMATIONEN AUF GRUNDLAGE DER AUSWAHL VON BAYESSCHEN SPITZENWERTEN UND ÜBERWACHUNGSVORRICHTUNGEN HIERMIT

PROCÉDÉS DE DÉTERMINATION D'INFORMATIONS PHYSIOLOGIQUES SUR LA BASE DE SÉLECTION DE PIC BAYÉSIEN ET DISPOSITIFS DE SURVEILLANCE LES INCORPORANT

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **29.12.2017　US 201762611764 P**

(43) Date of publication of application:
**16.09.2020　Bulletin 2020/38**

(73) Proprietor: **Yukka Magic LLC**
**Wilmington DE 19801 (US)**

(72) Inventors:
• **LEBOEUF, Steven Francis**
**Raleigh, North Carolina 27603 (US)**
• **HODGES, Ryan David**
**Raleigh, North Carolina 27604 (US)**
• **TANK, Tushar Dilip**
**Raleigh, North Carolina 27613 (US)**

(74) Representative: **Bardehle Pagenberg**
**Partnerschaft mbB**
**Patentanwälte Rechtsanwälte**
**Prinzregentenplatz 7**
**81675 München (DE)**

(56) References cited:
| | |
|---|---|
| US-A1- 2002 099 280 | US-A1- 2009 326 349 |
| US-A1- 2010 286 532 | US-A1- 2015 374 240 |
| US-A1- 2015 374 240 | US-A1- 2016 338 640 |
| US-A1- 2017 020 398 | US-A1- 2017 071 487 |
| US-A1- 2017 071 487 | |

• **SRINIVAS KUNTAMALLA ET AL: "An Efficient and Automatic Systolic Peak Detection Algorithm for Photoplethysmographic Signals",** INTERNATIONAL JOURNAL OF COMPUTER APPLICATIONS, vol. 97, no. 19, 1 July 2014 (2014-07-01), pages 18 - 23, XP055412228, DOI: 10.5120/17115-7686

**(Cont. next page)**

(52) Cooperative Patent Classification (CPC): (Cont.)
A61B 5/7257; A61B 2562/0219

**Description**

CLAIM OF PRIORITY

This application claims priority from U.S. Provisional Patent Application No. 62/611,764 filed on December 29, 2017 with the United States Patent and Trademark Office.

FIELD

The present invention relates generally to monitoring devices and, more particularly, to monitoring devices for measuring physiological information.

BACKGROUND

Wearable devices capable of monitoring physiological information, such as heart rate, are increasingly being used. These devices come in various form factors, including devices configured to be worn at the ear, wrist, or at other locations of the body. U.S. Patent Nos. 8,652,040, 8,700,111, 8,647,270, 8,788,002, 8,886,269, and 8,929,965 describe various wearable devices configured to monitor physiological information, including headsets, earbuds, and wrist bands.

Physiological information obtained from a subject can be used to generate various types of health and fitness assessments of the subject. For example, using a photoplethysmography (PPG) sensor incorporated into a wearable monitoring device, blood flow information can be measured during daily activities of a subject and this information can be used to generate assessments, such as maximum oxygen consumption $VO_2$max, total energy expenditure (TEE), etc.

Photoplethysmography (PPG) is based upon shining light into the human body and measuring how the scattered light intensity changes with each pulse of blood flow. The scattered light intensity will change in time with respect to changes in blood flow or blood opacity associated with heart beats, breaths, blood oxygen level ($SpO_2$), and the like. Such a sensing methodology may require the magnitude of light energy reaching the volume of flesh being interrogated to be steady and consistent so that small changes in the quantity of scattered photons can be attributed to varying blood flow.

However, if the incidental and scattered photon count magnitude changes due to light coupling variation between the source or detector and the skin or other body tissue, then the signal of interest can be difficult to ascertain due to large photon count variability caused by motion artifacts. Changes in the surface area (and volume) of skin or other body tissue being impacted with photons, or varying skin surface curvature reflecting significant portions of the photons may also significantly impact optical coupling efficiency. Physical activity, such as walking, cycling, running, etc., may cause motion artifacts in the optical scatter signal from the body, and time-varying changes in photon intensity due to motion artifacts may obscure time-varying changes in photon intensity due to blood flow changes. Environmental artifacts, such as ambient light noise, as well as motion-coupled ambient light noise can further obscure blood-flow related signals. Each of these changes in optical coupling can dramatically reduce the signal-to-noise ratio (S/N) of biometric PPG information to total time-varying photonic interrogation count. This can result in a much lower accuracy in metrics derived from PPG data, such as heart rate and breathing rate. When a PPG sensor is integrated into wearable devices used for daily living and exercise, motion artifacts and other noise sources can cause inaccurate heart rate readings and can destroy the possibility of accurate RR-interval (RRi) measurements. The article by Srinivas Kuntamalla et al." An Efficient and Automatic Systolic Peak Detection Algorithm for Photoplethysmographic Signals", International Journal of Computer Applications, vol. 97, no. 19, 1 July 2014, pages 18-23, XP055412228, describes an algorithm for eliminating minor peaks for PPG signals. US 2015/374240 by Lee Yong Jin/ Salutron, Inc. shows a motion sensor used to supplement PPG signals. US 2017/071487 by Ritscher et al./ Whoop Inc. has a similar aim; and reference may also be made to US 2010/2865532 (Farringdon et al./ Bodymedia, Inc.), and to US 2009/326349 (McGonigle et al.).

SUMMARY

The invention is defined in the independent claims. According to some embodiments of the present disclosure, a physiological signal processing method includes executing, by at least one processor, computer program instructions stored in a non-transitory computer readable medium. When executed, the computer program instructions cause the processor to perform operations comprising detecting respective peaks in a physiological waveform that represents physiological information collected from a subject over a period of time, computing probabilities for the respective peaks based on predetermined data indicative of one or more conditions, selecting a subset of the respective peaks based on the probabilities thereof as representing more accurate physiological information for the subject, and generating a physiological assessment of the subject based on the subset of the respective peaks that was selected. The physiological information is collected via at least one wearable device that comprises at least one physiological sensor and is worn by the subject.

In some embodiments, selecting the subset of the respective peaks may include determining combinations comprising sequences of peaks among the respective peaks over the period of time, and identifying one of the combinations based on a sum of the probabilities of the sequences of peaks thereof as the subset.

In some embodiments, at least some of the sequences of peaks may be non-consecutive peaks.

In some embodiments, the predetermined data may be received from one or more sensors that are distinct from the at least one physiological sensor. For example, the one or more sensors may be one or more optical sensors and/or motion sensors.

In some embodiments, the predetermined data may be derived from the physiological waveform.

In some embodiments, the physiological waveform may be a photoplethysmogram (PPG) signal, and the predetermined data may be a heart rate value, motion data detected by an accelerometer, and/or energy response signal data.

In some embodiments, the more accurate physiological information may be an R-R time-series including consecutive R-R intervals therein. Generating the physiological assessment may further include determining whether a heart rate variability metric for the subject is within a predetermined range, where the heart rate variability metric may be calculated based on a group of the consecutive R-R intervals for the subject.

In some embodiments, the data indicative of the predetermined data may be a heart rate value generated based on frequency domain analysis different from that used to provide the R-R time-series.

In some embodiments, computing the probabilities may include computing initial probabilities for the respective peaks based on amplitudes thereof, and computing weighted or normalized probabilities for the respective peaks based on the amplitudes thereof relative to adjacent peaks of the respective peaks.

In some embodiments, computing the probabilities may further include computing probabilities for respective intervals that include two or more of the respective peaks, where the respective intervals occur over the period of time, and determining the probabilities based on the weighted or normalized probabilities for the respective peaks and the probabilities for the respective intervals.

In some embodiments, the weighted or normalized probabilities are based on a Gaussian distribution.

In some embodiments, the physiological waveform is a time-domain representation or a frequency-domain representation.

In some embodiments, the wearable device may be an earbud, an audio headset, a wrist strap, a wrist watch, an ankle bracelet, or an armband, and the at least one physiological sensor may be part of a biometric monitoring device that is integrated within the wearable device.

According to some embodiments of the present disclosure, a wearable device includes at least one physiological sensor configured to detect and/or measure physiological information from a subject over a period of time when the wearable device is worn by the subject, and a processor coupled to the sensor. The processor is configured to detect respective peaks in a physiological waveform representing the physiological information, compute probabilities for the respective peaks based on predetermined data indicative of one or more conditions, select a subset of the respective peaks based on the probabilities thereof as representing more accurate physiological information for the subject, and generate a physiological assessment of the subject based on the subset of the respective peaks that was selected.

In some embodiments, the processor may be configured to select the subset of the respective peaks by determining combinations comprising sequences of peaks among the respective peaks over the period of time, and identifying one of the combinations based on a sum of the probabilities of the sequences of peaks thereof as the subset.

In some embodiments, at least some of the sequences of peaks may be non-consecutive peaks.

In some embodiments, the wearable device may further include one or more sensors that are distinct from the at least one physiological sensor, and the predetermined data may be received from the one or more sensors. For example, the one or more sensors may be one or more optical sensors and/or motion sensors.

In some embodiments, the predetermined data may be derived from the physiological waveform.

In some embodiments, the physiological waveform may be a photoplethysmogram (PPG) signal, and the predetermined data may be a heart rate value, motion data detected by an accelerometer, and/or energy response signal data.

In some embodiments, the more accurate physiological information may be an R-R time-series including consecutive R-R intervals therein, and the processor may be configured to generate the physiological assessment by determining whether a heart rate variability metric for the subject is within a predetermined range. The heart rate variability metric may be calculated based on a group of the consecutive R-R intervals for the subject.

In some embodiments, the data indicative of the predetermined data may be a heart rate value generated based on frequency domain analysis different from that used to provide the R-R time-series.

In some embodiments, the processor may be configured to determine the probabilities by computing initial probabilities for the respective peaks based on amplitudes thereof, and computing weighted or normalized probabilities for the respective peaks based on the amplitudes thereof relative to adjacent peaks of the respective peaks.

In some embodiments, the processor may be further configured to determine the probabilities by computing probabilities for respective intervals that include two or more of the respective peaks, wherein the respective intervals occur over the period of time, and determining the probabilities based on the weighted or normalized probabilities for the respective peaks and the probabilities for the respective intervals.

According to some embodiments of the present disclosure, a physiological signal processing device includes an electronic circuit comprising a non-transitory computer readable medium having program instructions stored therein, and at least one processor that is configured to execute the computer program instructions stored in the non-transitory computer readable medium. When executed, the program instructions cause the processor to perform operations comprising detecting respective peaks in a physiological waveform that represents physiological information collected from a subject over a period of time, computing probabilities for the respective peaks based on predetermined data indicative of one or more conditions, selecting a subset of the respective peaks based on the probabilities thereof as representing more accurate physiological information for the subject, and generating a physiological assessment of the subject based on the subset of the respective peaks that was selected. The physiological information is collected via at least one wearable device that comprises at least one physiological sensor and is worn by the subject.

According to some embodiments of the present disclosure, a computer program product for physiological signal processing includes a non-transitory computer readable medium having computer program instructions stored therein. When executed by at least one processor, the computer program instructions cause the at least one processor to perform operations comprising detecting respective peaks in a physiological waveform that represents physiological information collected from a subject over a period of time, computing probabilities for the respective peaks based on predetermined data indicative of one or more conditions, selecting a subset of the respective peaks based on the probabilities thereof as representing more accurate physiological information for the subject, and generating a physiological assessment of the subject based on the subset of the respective peaks that was selected. The physiological information is collected via at least one wearable device that comprises at least one physiological sensor and is worn by the subject.

In some embodiments, selecting the subset of the respective peaks may include determining combinations comprising sequences of peaks among the respective peaks over the period of time, and identifying one of the combinations based on a sum of the probabilities of the sequences of peaks thereof as the subset.

In some embodiments, at least some of the sequences of peaks may be non-consecutive peaks.

In some embodiments, computing the probabilities may include computing initial probabilities for the respective peaks based on amplitudes thereof, and computing weighted or normalized probabilities for the respective peaks based on the amplitudes thereof relative to adjacent peaks of the respective peaks.

In some embodiments, computing the probabilities may further include computing probabilities for respective intervals that include two or more of the respective peaks, wherein the respective intervals occur over the period of time, and determining the probabilities based on the weighted or normalized probabilities for the respective peaks and the probabilities for the respective intervals.

BRIEF DESCRIPTION OF THE DRAWINGS

FIG. 1 is a block diagram illustrating a physiological signal processing system according to some embodiments of the present disclosure.

FIG. 2 is a graph illustrating operations for determining and assigning probabilities to respective peaks in a filtered section of a physiological waveform in accordance with some embodiments of the present disclosure.

FIG. 3 is a graph illustrating possible connections between the respective peaks of a probability matrix generated in accordance with some embodiments of the present disclosure.

FIG. 4 is a graph illustrating selection of multiple subsets of peaks, among the possible connections between the respective peaks of a probability matrix in accordance with some embodiments of the present disclosure.

FIG. 5A is a block diagram illustrating an example signal processing device in accordance with some embodiments of the present disclosure.

FIG. 5B is a flowchart illustrating example operations that may be performed by a signal processing device in accordance with some embodiments of the present disclosure.

FIGS. 6A-6B are graphs illustrating an RRi waveform output prior to (FIG. 6A) and responsive to (FIG. 6B) operations in accordance with some embodiments of the present disclosure, as compared to the output of a chest strap heart monitor.

FIGS. 7A-7B and 8A-8B illustrate example wearable devices that may incorporate sensor systems in accordance with some embodiments of the present disclosure.

DETAILED DESCRIPTION OF EMBODIMENTS

Some embodiments of the present disclosure may arise from realization that noise and/or other artifacts that may be present in a physiological signal or waveform may obscure desired physiological information that may be included in or derived from the waveform. For example, for a photoplethysmography (PPG) signal output from a PPG sensor, heart rate variability (HRV) metrics can be calculated from the RR-interval time series, which may require accurate identification of the respective locations (over the time period of measurement) of the peaks associated with blood flow of the PPG signal. However, electrical and mechanical noise, respiration, motion artifacts, etc., can contribute to noise on a PPG signal, and can thus create multiple peaks in the output signal, or can destroy peaks or data integrity, which can obscure and/or be confused with the desired peaks associated with blood flow.

Embodiments described herein provide methods, systems, and wearable devices that utilize a priori knowledge, i.e., predetermined data indicative of information or conditions, to determine and assign probabilities of validity to each peak in a physiological signal or waveform (for example, as output from a PPG or other physiological sensor), and select a subset including a combination of the respective peaks (e.g., a more probable combination of the peaks) as a more accurate representation of the physiological information that is present in the physiological waveform, based on the determined probabilities. For example, the selected subset may include peaks from the waveform that are more likely to accurately represent a PPG RR-interval time series. The predetermined data is non-static and may include, but is not limited to, heart rate, accelerometer data, data derived from the waveform (e.g., a slope of the PPG signal), empirically fitted distributions, RR-interval(s), respiration rate, etc., and/or combinations thereof. That is, the predetermined data can be provided by one or more sensors that are distinct from the physiological sensor (regardless of whether the sensors are worn, portable, or remote) that provides the physiological signal, and/or can be derived from the physiological signal itself. Particular embodiments described herein calculate the probability of each possible peak combination based on such predetermined data of related conditions to create a Bayesian framework, and select the peak combination which has a higher (or the highest) probability of being associated with blood flow of a PPG signal (or other desired physiological information in a waveform), effectively removing or filtering out peaks that may be attributed to noise or are otherwise inaccurate with respect to the desired physiological information contained in the waveform.

It will be understood that the predetermined data can include a priori factors that may be unknown and conditioned on other a priori knowledge. That is, the predetermined data may be used to create the Bayesian framework as a chain of Bayesian conditionals, which may be desirable due to the complexity of the human body. For example, given a heart rate (HR), a probability of Peaks, P[p|HR], can be created; however, as the HR may not be known exactly, the HR can be conditioned on other factors (e.g., activity level (AL), such that P[p|HR]*P[HR|AL]*P[AL]).

As described in greater detail below, embodiments of the present disclosure may be used alone or in combination with additional operations for motion artifact removal, increasing both accuracy (by using multiple noise removal operations) and robustness (by using orthogonal noise removal operations). Although described herein primarily with reference to time-domain waveforms based on output from a PPG sensor, it will be understood that the operations for peak detection, probability assignment, and subset selection described herein are not so limited, and can be applied to other waveforms (e.g., frequency-domain waveforms/spectral analysis) to similarly identify peaks or peak combinations that more accurately represent the desired physiological information (also referred to herein as "valid" peaks or peak combinations) and generate a physiological assessment of the subject based thereon. In addition, the method may be applied to ECG waveforms, auscultatory waveforms (acoustic waveforms from the body), ballistocardiogram waveforms, and the like. Moreover, the method may be applied to non-heart-rate waveforms of the PPG signal, such as respiration waveforms within the PPG signal, or other periodic or quasi-periodic information within the PPG signal. Additionally, the probability information may be stored as historical data in a memory device, and can be used to assess medical conditions based on information which might otherwise be inadvertently filtered out of the physiological signal. For example, lower probability peaks may be stored and analyzed for use in a subsequent physiological assessment (e.g., extended durations with low probabilities may indicate a cardiac condition) and/or for selection or modification of one or more thresholds for the present or subsequent physiological assessment.

The term "RRi" refers to the "R-R interval" which is the time interval between consecutive R-wave peaks seen on an electrocardiogram (ECG), and when used in embodiments of the present disclosure, may further include the series of intervals between peaks due to blood flow in a photoplethysmogram (PPG) of a subject (often called "PPi" or "pulse-to-pulse interval"). Generally, where heart rate is used in embodiments of the present disclosure, RRi may also be applied in a similar manner. However, RRi and heart rate are generally related in an inverse fashion, such that $1/RRi = $ instantaneous heart rate.

The term "HRV" refers to "heart rate variability" or "R-R variability", which is a statistical representation of a group of consecutive R-R intervals or N-N intervals (beat-to-beat intervals between heart beats). The types of statistics performed to generate an HRV value can be quite numerous and broad. In general, a variety of different time-domain and/or frequency domain statistics on heart beat intervals can be described as different HRV values. As one specific example of HRV, 2- or 5-minutes worth of R-R intervals may be processed to determine the mean (AVNN) and standard deviation (SDNN), which are representations of HRV. In general, the higher the SDNN for a group of R-R intervals collected from a person, the more relaxed, physically fit, attentive, ready for exercise, or healthy that person may be. N-N intervals may be collected via photoplethysmograms (PPG), electrocardiograms (ECG), blood pressure pulses, ballistocardiograms (BCG), and the like.

In the following figures, various monitoring devices will be illustrated and described for attachment to the wrist or ear of the human body. However, it is to be understood that embodiments of the present invention are not limited to the illustrated monitoring devices or to those worn by humans. For example, embodiments of the present invention may be integrated into clothing, apparel, jewelry (such as finger rings, earrings, pendants, necklaces, etc.), video cameras or imaging sensors, leg bands, patches, and the like.

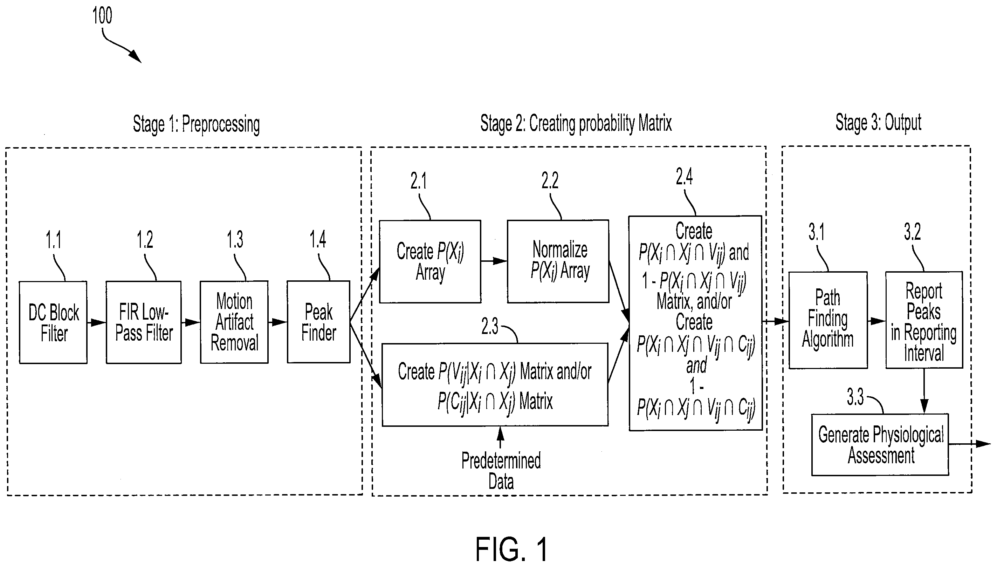

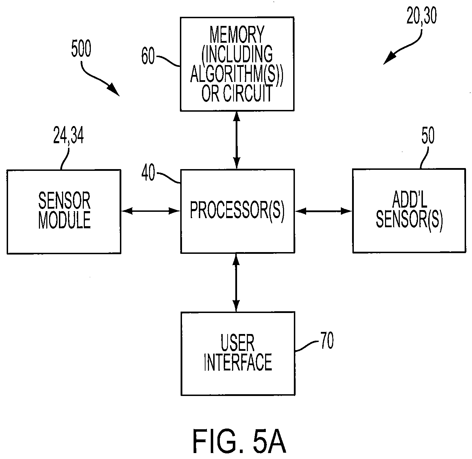

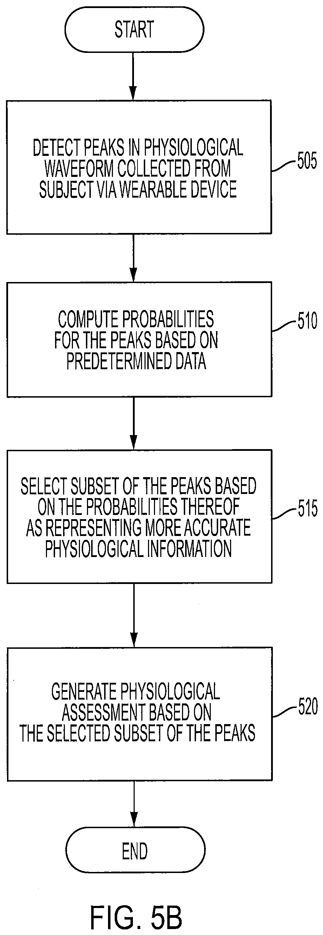

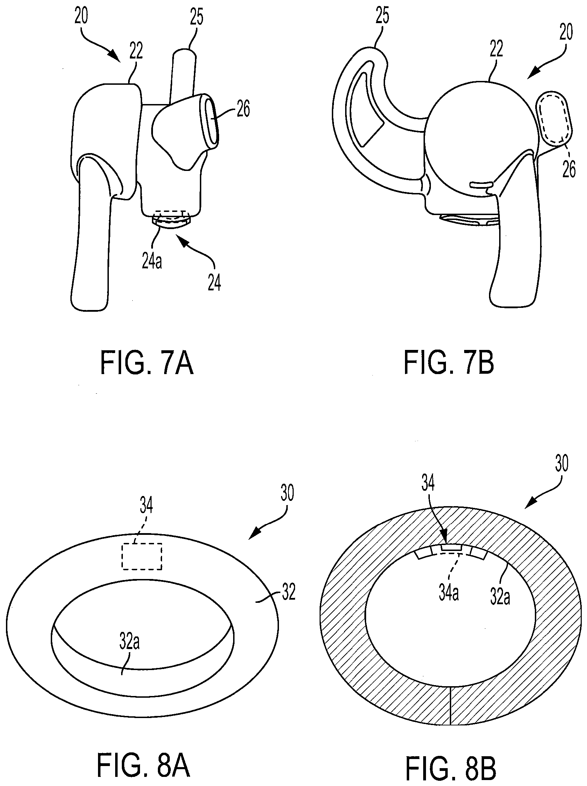

FIG. 1 is a block diagram illustrating a physiological signal processing system according to some embodiments of the present disclosure. The system 100 is described herein with reference to three stages (Stage 1, Stage 2, Stage 3); however, it will be understood that fewer or additional stages may be included in some embodiments. The operations performed by the system 100 of FIG. 1 may be implemented by at least one processor circuit (such as the processor 40 of FIG. 5), which may include microprocessors, microcontrollers, ASICs (application specific integrated circuit), analog processing circuitry, digital signal processors, optical circuitry, magnetic circuitry, neural processor circuitry, or the like. In some embodiments, each block shown in FIG. 1 may represent readable program code stored in a non-transitory memory device (such as the memory device 60 of FIG. 5) that is coupled to the processor, such that the processor may execute the computer readable program code represented by the blocks to perform the operations described with reference to FIG. 1. The processing circuit may be at least partially implemented in one or more monitoring devices (such as the monitoring devices 20, 30 of FIGS. 7A-7B and 8A-8B) as described herein.

Referring now to FIG. 1, Stage 1 illustrates example preprocessing circuits and operations in accordance with some embodiments of the present disclosure. In particular, Blocks 1.1 and 1.2 provide circuits and related operations configured to digitally filter and preprocess a raw physiological signal or waveform (such as a PPG signal) that is output from a physiological sensor. The output from the physiological sensor thus includes physiological information collected from a subject over a period of time via at least one wearable device (such as the monitoring devices 20, 30), and is stored in a memory device (such as the memory device 60 of FIG. 5) coupled to the processor. The operations include DC blocking in Block 1.1, and FIR (finite impulse response) and/or IIR (infinite impulse response) low-pass, high-pass, and band-pass filtering in Block 1.2.

Block 1.3 provides circuits and related operations configured to remove motion artifacts in the physiological signal or waveform, for example, using a noise reference (such as a motion sensor, i.e. an accelerometer or the like). The operations include, but are not limited to, time domain methods such as adaptive filters, frequency domain methods such as spectral transforms (such as an FFT (fast Fourier transform) or the like), and/or eigen-decomposition methods (such as MUSIC (Multiple Signal Classification)). More generally, the operations of Block 1.3 may use the above and/or other circuits and operations to remove and/or diminish motion artifacts in the physiological signal or waveform, and present a "cleaner" signal or waveform to Block 1.4. In particular, Block 1.4 provides circuits and related operations for detecting or otherwise identifying some or all of the peaks (e.g., based on local maxima and minima) of a filtered and preprocessed PPG waveform that is output from Block 1.3. In a particular non-limiting example, zero crossings of the derivative may be used for peak detection. Additional or alternative non-limiting examples of preprocessing methods for PPG waveforms may include those described in U.S. Patents 9,801,552, 8,923,941, and 8,512,242.

Still referring to FIG. 1, Stage 2 illustrates example circuits and operations configured to determine and assign a respective probability to each of the peaks that were detected or otherwise identified in Block 1.4 in accordance with some embodiments of the present disclosure. For example, Stage 2 may provide circuits and operations configured to create a probability matrix representing the peaks detected in the input waveform.

In particular, Blocks 2.1 and 2.2 provide circuits and related operations configured to use the amplitude of each peak to create and normalize a probability array $P(X_i)$, which is a N x 1 array where N indicates the total number of peaks in the sampled time period or reporting window. Block 2.1 defines initial probabilities based on local peak amplitude (for example, larger peaks may be assigned higher probabilities). Block 2.2 uses a Gaussian PDF (probability density function) to perform a weighted average of the initial probabilities from Block 2.1, based on the proximity of other peaks relative to the local peak amplitude (i.e., the mean peak value $\mu$ stretched by the standard deviation $\sigma$).

In some embodiments, a detected peak may be associated with a higher probability of being a valid peak if it has a comparatively large amplitude and is the only local maximum nearby (e.g., is not in close proximity to other peaks), and may be associated with a lower probability of being a valid peak if it has a comparatively smaller amplitude and several local maxima nearby. In this context, the term "valid peak" means that the peak of the waveform corresponds to the underlying physiological phenomenon that the waveform is attempting to capture. For example, the peak in the waveform could correspond to an individual heart beat. Any "nearby" peaks in close proximity may be dynamically determined and/or altered; that is, the time period (or waveform portion) used to determine what is defined as "close proximity" may not correspond to a static period of time or portion of the waveform, but may dynamically vary based on the distribution of peaks. Thus, the initial probabilities determined based on the amplitude values of the detected peaks at Block 2.1 may be weighted or normalized based on proximity of other peaks at Block 2.2. Although described herein primarily with reference to Gaussian PDFs, it will be understood that embodiments of the present disclosure are not so limited, and that other probability distributions may be used to determine and assign probabilities to peaks and/or other portions of a physiological waveform in accordance with embodiments of the present disclosure.

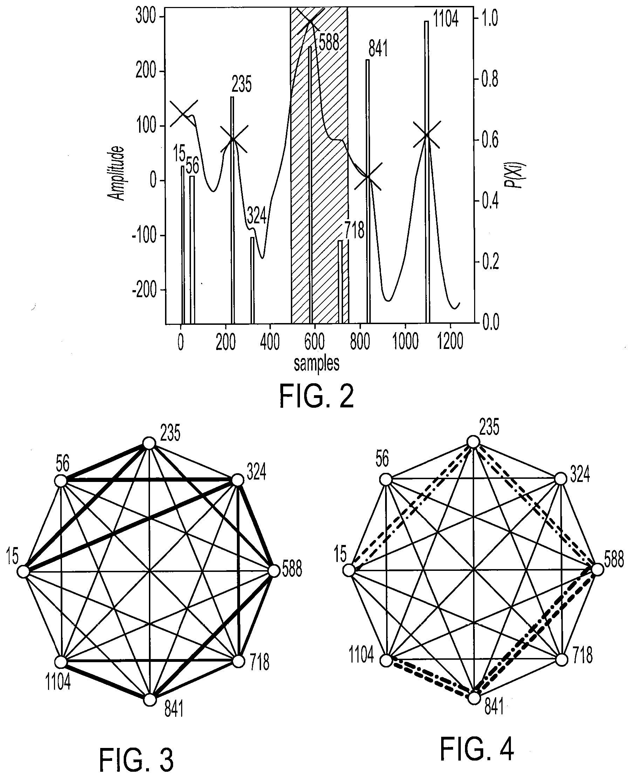

FIG. 2 is a graph further illustrating operations for identifying peaks and determining and assigning respective probabilities to the peaks in a filtered section of a physiological waveform in accordance with some embodiments of the present disclosure. As shown in FIG. 2, the bars associated with each peak represent the normalized probability that each specific peak is considered a valid peak based on the operations of Blocks 2.1 and 2.2 discussed above.

Returning to FIG. 1, Block 2.3 provides circuits and related operations configured to create a probability matrix $P(V_{ij}|X_i \cap X_j)$, which is an N x N matrix where each position i, j contains the probability $V_{ij}$ that an interval including peaks $X_i$ and $X_j$ is a valid interval based on the expected time between intervals. In some embodiments, Block 2.3 may further create additional conditional probability matrices such as $P(C_{ij}|X_i \cap X_j)$, which is an N x N matrix where each position i, j contains the probability $C_{ij}$ that the section of the PPG signal containing $X_i$ and $X_j$ is clean, i.e. free from motion artifacts, based on a priori knowledge from predetermined data (e.g. accelerometer data). For example, an output signal from an accelerometer and/or other motion sensors may indicate whether and/or how the subject is moving (e.g., by indicating periodic or aperiodic motion, for example, as described in U.S. Patent Application Publication No. 2017/0112447 to Aumer et al.), which may be analyzed to increase or decrease a probability that detected peaks in an interval of time corresponding to the detected motion are valid. For example, if periodic motion is detected by processing the output of an accelerometer, then PPG peaks that fall within the period of motion may be assigned a lower probability than PPG peaks that fall outside (or substantially outside) of that period. In the case when the period of motion is equal or substantially equal to the period of the heartrate, it may be identified that the user is in a crossover state (where the peak heart rate frequency and peak motion frequency are the same or nearly the same), and the probability constraints may be modified (such that if PPG peaks fall within the period of motion, it will not substantially reduce the probability that the PPG peaks represent heartbeats).

As such, embodiments described herein may generate probability matrices based on the probability of validity of respective peaks X ($P(X_i)$ and $P(X_j)$), the probability of validity of intervals including each peak ($P(V_{ij})$), the probability that the signal containing each peak is clean ($P(C_{ij})$), and/or additional conditional probabilities. The matrix $P(V_{ij})$ can be calculated, for example, by sampling from a Gaussian PDF of the probability of expected intervals based on the known heart rate value or other predetermined data which may increase or decrease the likelihood that a particular interval, or other portion of the waveform is valid based on the timing between peaks. For example, the known heart rate value may be generated by processing the PPG waveform using a variety of methods. Non-limiting examples of methods for calculating the heart rate value may include those described in U.S. Patent No. 8,923,941 and U.S. Patent Publication No. 2015/0018636. The matrix $P(C_{ij})$, can be calculated, for example, by sampling from an empirically derived PDF of the probability of corrupting motion artifacts based on the known accelerometer value or other predetermined data which may increase or decrease the likelihood that a particular interval, or other portion of the waveform, is attributable to noise. The predetermined data (whether from the waveform itself, another sensor, or other information source) may thus be used as additional factor(s) that affect the overall likelihood of two peaks defining a valid interval, to remove or filter out peaks that may be attributed to noise or are otherwise inaccurate with respect to the desired physiological information contained in the waveform.

FIG. 3 is a graph illustrating possible connections between the peaks X of the probability matrix generated at Block 2.3. In particular, the graph of FIG. 3 illustrates the probability that each i, j pair of peaks X together create a valid interval, that is, $P(V_{ij}|X_i \cap X_j)$. In the graph of FIG 3, each node represents a detected peak, and the width of the connection between each node (i.e., the width or thickness of the connecting segments between nodes) represents the probability that the two nodes create a valid interval. That is, in the example of FIG. 3, nodes with thicker connecting segments therebetween may represent a higher-probability sequence of peaks.

Returning to FIG. 1, Block 2.4 provides circuits and related operations configured to use the $P(X_i)$ array (output from Blocks 2.1 and 2.2) and the $P(V_{ij}|X_i \cap X_j)$ matrix (output from Block 2.3) to create the $P(X_i \cap X_j \cap V_{ij})$ and $1 - P(X_i \cap X_j \cap V_{ij})$ matrix, where $P(X_i \cap X_j \cap V_{ij}) = P(X_i) P(X_j)P(V_{ij}|X_i \cap X_j)$, assuming independence (naïve Bayes). These matrices contain the probability (or "not" probability, respectively) that each i, j pair of peaks X are valid peaks that together create a valid interval. In this context, the term "valid interval" means that the i and j peaks represent a physiologically significant pair. For example, the ith peak and the jth peak may represent consecutive heart beats and thus the time between the peaks represents the interval between heart beats. In some embodiments, the circuits and operations of Block 2.4 may further use the $P(C_{ij}|X_i \cap X_j)$ matrix to create the $P(X_i \cap X_j \cap V_{ij} \cap C_{ij})$ and $1 - P(X_i \cap X_j \cap V_{ij} \cap C_{ij})$ matrix, which contain the probability (or "not" probability, respectively) that each i, j pair of peaks X are valid peaks, that the peaks together create a valid interval, and that intervals between the peaks are clean. The circuits and operations of Block 2.4 can be extended to include similar matrices for any number of attributes, sensor data, or external information that may affect the probability that a combination of two peaks creates a physiologically significant pair or valid interval. Generally, the chain rule in probability theory

$$P(\cap_{k=1}^{n} A_k) = \prod_{k=1}^{n} P(A_k \mid \cap_{j=1}^{k-1} A_j)$$ can be

applied to include any other signal, sensor, or information that may affect the probability of each potential peak pairing.

Still referring to FIG. 1, Stage 3 illustrates example circuits and operations for selecting a combination of the peaks that provide a more accurate representation of the physiological information included in the physiological waveform in accordance with some embodiments of the present disclosure. As shown in FIG. 4, once the probability matrices $P(X_i \cap X_j \cap V_{ij})$ and $1 - P(X_i \cap X_j \cap V_{ij})$ are calculated in Stage 2, Block 3.1 provides circuits and related operations that select a subset of the peaks that either increase/maximize the probability (or reduce/minimize the not probability) that the peaks are valid. In this example, Block 3.1 selects the subset by traversing the possible paths (e.g., sequences of peaks) represented by the graph shown in FIG. 4, and finding either the longest/maximum path through $P(X_i \cap X_j \cap V_{ij})$ or the shortest/minimum path through $1 - P(X_i \cap X_j \cap V_{ij})$, as shown in FIG. 4.

FIG. 4 is a graph illustrating selection of multiple subsets of peaks, among the possible connections between the peaks X shown in FIG. 2, where the peaks marked with an "X" in FIG. 2 are considered to be valid and those not marked with an "X" are considered to be invalid based on the operations of Block 3.1. In particular, the graph of FIG. 4 illustrates the probability that each i, j pair of peaks X are valid peaks and together create a valid interval, that is, $P(X_i \cap X_j \cap V_{ij})$. The peaks in the final selected path (shown in dashed lines, with thicker lines representing higher probability) are considered to be the most probable sequence of peaks in the waveform, that is, a combination of peaks that more accurately represents the measured physiological information collected from the subject. For example, one method for selection of the peaks is to use Dijkstra's path finding algorithm to find the shortest path through $1 - P(X_i \cap X_j \cap V_{ij})$. However, it will be understood that embodiments of the present disclosure are not so limited, and that other path finding methods may be used. Likewise, the graphs shown in FIGS. 3 and 4 are provided by way of example and embodiments of the present disclosure are not so limited, and other types of graphs (e.g., directed acrylic graphs) can be used to model the peaks and possible paths as described herein.

Returning to FIG. 1, Block 3.2 provides circuits and related operations configured to generate an output including the selected subset of peaks (where the time between two peaks is an interval) in near real-time or in post processing, for example, for reporting to an end user and/or for generation of a physiological assessment of the subject (in Block 3.3). The output may include all peaks in the reporting window in some embodiments. Each peak may also be flagged based on the actual probability associated therewith, and a threshold may be applied such that only peaks (or intervals or paths associated with peaks) associated with probabilities above the threshold will be used in the final R-R time series. Moreover, this probability may be stored in a memory buffer, along with the respective peak or R-R interval, to be later used in additional physiological assessments. For example, a consistently low probability that a peak and/or the best path is valid may indicate that the subject is suffering from a medical condition, such as an arrhythmia, atrial fibrillation, or other cardiovascular abnormality. In this way, associating the peak or R-R interval (or other calculated interval) with the respective probability, and storing these values in a buffer for transmission to a remote device, can be used for a variety of health and fitness assessments. Thus, the probabilities may be used not just for picking or selecting the best or more accurate path (for generating RR-intervals) but also for generating health physiological assessments (such as a notification of arrhythmia or abnormal heart beats).

Block 3.3 provides circuits and related operations configured to generate a physiological assessment of the subject based on the selected subset of peaks, which more accurately represent the measured or collected physiological information. For example, embodiments of the present disclosure can be used either in near real-time or in post processing to generate an accurate R-R time-series from a PPG signal, and the R-R time series can be used to calculate accurate HRV metrics which are used to assess training effectiveness, controlled breathing, certain types of arrhythmia, etc. In particular, arrhythmia (such as atrial fibrillation) and other cardiac conditions may be detected by leveraging the probability information that is generated for each RRi estimate; that is, if the probabilities are low for an extended duration of time, this may indicate that a cardiac condition may be present.

In addition, more accurate RRi and HRV metrics as generated in accordance with embodiments of the present disclosure can be used to assess and/or track sleep, stress, exercise, etc. For example, it may be recognized that a PPG waveform has a lower variability at high heart rates, and a greater variability at lower heart rates; thus, the lower variability may be an indicator that the subject is exercising, recovering from strenuous activity, stressed, fatigued, or in a state of being less alert, while the greater variability may be an indicator that the subject is resting, relaxed, recharged, or in a state of relative high alertness.

In some embodiments, the a priori or predetermined information may be detected or derived from optical sensor outputs. For example, one or more optical sensors may be configured to emit light in multiple different wavelength ranges, and to detect an energy response signal that includes the multiple wavelengths. In particular, PPG signals generated in response to emission of multiple wavelengths of light can be used to improve the probability distributions. For example, light in an optical wavelength range (wavelength 2) that is more sensitive to motion artifacts (e.g., an infrared wavelength range optical emitter in a wrist-based PPG device) may be expected to generate a larger magnitude in a "fake PPG peak" caused by motion when compared to that of an optical wavelength range (wavelength 1) that is less sensitive to motion artifacts (e.g., a green wavelength range optical emitter in a wrist-based PPG device). Thus, if a neighboring peak from wavelength 2 PPG output shows an increase in peak amplitude while the corresponding neighboring peak from wavelength 1 PPG output does not show in an increase in the peak amplitude, then the probability that the neighboring peaks represent a valid RR-interval may be lowered based on the inconsistency. It should be noted that the choice in optical wavelength for wavelength 1 and wavelength 2 may be body location-dependent and even subject-dependent. For example, in the ear, infrared wavelengths may cause less motion artifacts (be more motion-tolerant) than green wavelengths, and a subject having darker skin tone may have more motion-tolerant readings responsive to emission of infrared wavelengths at the wrist than with a shorter wavelength such as green, blue, or violet. Similarly, if multiple emitters and/or multiple detectors are arranged within a PPG sensor, where there are a plurality of optical paths between plurality of emitter-detector configurations, then with alternating biasing in time, various optical paths can be sampled in a short period of time ($\Delta t_{sample}$) corresponding to the time interval of at least one peak, enabling a plurality of peaks to be evaluated for generating one overall peak for use in the $P(X_i)$ array. In this context, an optical path refers to a physical path taken by a beam of light from the respective emitter to the respective detector. As a specific example, in an arrangement with a plurality of optical emitters and one optical detector (thus a plurality of optical paths corresponding to at least one path for each emitter with respect to the single detector), each optical emitter may be alternately biased in time at a given frequency f (or over a period p). Thus, in one sample period ($\Delta t_{sample}$), where the sample period is notably smaller than (e.g., less than 1/10$^{th}$) the time of one heartbeat, a plurality of peaks for each of the optical paths may be assessed to generate an overall probability for the heartbeat waveform peak (which is represented by the plurality of peaks). Namely, the amplitudes of the plurality of peaks may be assessed to generate a probability for the overall heartbeat waveform peak to be used in the $P(X_i)$ array. For example, if two or more of the plurality of peaks are substantially different in amplitude or phase, or if one or more of the plurality peaks are missing, then the probability associated with the overall heartbeat waveform peak may be lower as used in the $P(X_i)$ array. That is, inconsistencies with respect to one or more peaks measured for each of the optical paths may indicate a lower probability that the one or more peaks are valid. A physiological reasoning for this is that dissimilar amplitudes, phase, etc. associated with the plurality of peaks could suggest that the overall heartbeat waveform peak detected is more likely associated with an artifact (such as a motion artifact or environmental artifact) than a true or valid heartbeat peak.

In some embodiments, the a priori or predetermined information may be detected or derived from outputs of one or more sensors that are distinct from the physiological sensor from which the physiological waveform is generated. For example, one or more motion sensors (such as an accelerometer) may be configured to generate a motion-based output signal, which may be processed to feed-in to the probability distribution estimation to increase accuracy. For instance, a motion-based output signal generated during random motion (such as associated with lifestyle activities) will differ from a motion-based output signal generated during periodic activities (such as exercise), and the probability that a peak or RR-interval is valid may be reduced when contemporaneous conditions of high or erratic (nonperiodic) motion or alternatively periodic motion are detected by the motion sensor.

Further embodiments may utilize a priori or predetermined information derived from outputs from multiple types of sensors (e.g. outputs of optical sensors responsive to one or more emission wavelengths in combination with outputs of accelerometers, skin-contact pressure sensors, and/or auscultatory sensors) for probability determination. For example, if a neighboring peak from wavelength 2 PPG output shows an increase in peak amplitude while the corresponding neighboring peak from wavelength 1 PPG output does not show in an increase in the peak amplitude, and if a motion sensor output signal also indicates erratic or nonperiodic motion in the interval between the neighboring peaks, then the probability that the corresponding RR-interval is valid may be further reduced. The a priori or predetermined information may include additional contextual information, which may be further applied to generate a physiological assessment. For example, a wearable device may include a proximity sensor (e.g., a skin contact sensor) that is configured to output a signal (e.g., a "being worn" flag) if the wearable device is currently being worn. If such a being worn flag is present, and if a motion assessment (for example, as determined based on an output of an accelerometer or other motion sensor) indicates that the person is not moving, and if the probabilities are still low for an extended duration of time (e.g., several minutes), an alert condition may be generated to indicate that a cardiac condition may be present.

FIG. 5A is a block diagram illustrating an example signal processing device 500 in accordance with embodiments described herein and FIG. 5B is a flowchart illustrating example operations that may be performed by a signal processing device in accordance with embodiments described herein, such as the device 500 of FIG. 5A. In some embodiments, the device 500 may be included in or otherwise in communication with a monitoring device (e.g., monitoring devices 20, 30 shown in FIGS. 7A-7B and 8A-8B). Referring to FIG. 5A, the illustrated device 500 includes a sensor module 24, 34 having one or more physiological sensors configured to detect and/or measure physiological information from the subject, and one or more additional sensors 50. In some embodiments, the physiological sensors may be optical sensors (each including at least one optical emitter and at least one optical detector) configured to detect optically derived physiological information from a location on a body of a subject.

The additional sensor(s) 50 are distinct from the physiological sensors of the module 24, 34, and are configured to detect one or more conditions and output predetermined data indicative thereof. The sensor(s) 50 may include, but are not limited to, one or more inertial sensors (e.g., an accelerometer, piezoelectric sensor, vibration sensor, photoreflector sensor, etc.) for detecting changes in motion, one or more thermal sensors (e.g., a thermopile, thermistor, resistor, etc.) for measuring temperature of a part of the body, one or more electrical sensors for measuring changes in electrical conduction, one or more skin humidity sensors, one or more optical sensors, and/or one or more acoustical sensors or auscultatory sensors. More generally, the additional sensor(s) 50 may be representative of a variety of sensor types from which the predetermined data for the probability determination described herein can be derived.

The signal processing device 500 also includes a non-transitory memory device 60 and at least one processor 40 coupled thereto. The processor 40 is communicatively coupled to the sensor(s) 24, 34, and 50, and is configured to receive and analyze signals produced by the sensor(s) to perform the operations illustrated in FIG. 5B. In particular, the processor 40 is configured to execute computer readable program code stored in the memory 60 to detect respective peaks in a physiological waveform that represents physiological information collected from a subject over a period of time via a physiological sensor (such as the sensor module 24, 34) of a wearable device (such as the monitoring devices 20,30) at block 505; compute probabilities for the respective peaks based on predetermined data indicative of one or more conditions (such as the data output from the sensor(s) 50) at block 510; select a subset of the respective peaks based on the probabilities thereof as representing more accurate physiological information for the subject at block 515; and generate a physiological assessment of the subject based on the more accurate physiological information at block 520, as described in greater detail above with reference to the example of FIG. 1. More generally, the processor 40 may represent electronic circuitry and/or combinations thereof that are configured to perform the operations described herein, including but not limited to a digital signal processor (DSP) or microcontroller, an integrated circuit or application-specific integrated circuit (ASIC), analog and/or digital gates such as field programmable gate arrays (FPGAs), and/or neural circuits, some of which may be configured to perform one or more of the operations with lower power consumption and/or higher speed.

It will be understood that the processor 40 and the sensor(s) 24, 34, and 50 need not be co-located in a common housing, and may be a remotely located in some embodiments. More generally, the connections illustrated by arrows between the elements shown in FIG. 5A may represent wired and/or wireless communication connections between the elements, and thus, one or more of the illustrated elements may be included in respective remote devices that are in wireless communication. As such, in some embodiments, the sensor(s) 50 may be included in a device that is external to a monitoring device that includes the physiological sensor module 24, 34. The processor 40 is further configured to generate a physiological assessment of the subject based on the operations described herein, and to transmit the physiological assessment to a user interface 70 for display thereon as an audio and/or visual representation of the assessment. It should be noted that the operations of described herein may be controlled by algorithms, circuitry, or a combination of both.

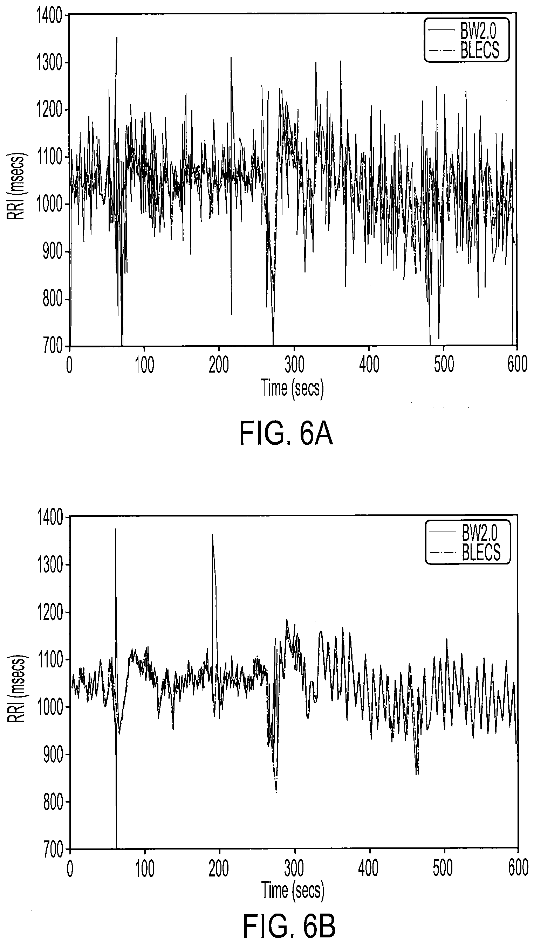

FIGS. 6A-6B are graphs illustrating an RRi waveform output prior to (FIG. 6A) and responsive to (FIG. 6B) operations in accordance with some embodiments of the present disclosure, as compared to the output of a chest strap heart monitor (BLECS). In FIG. 6A and FIG. 6B, the RRi waveforms are presented for both a BW2.0 unit, a wrist-worn sensor module developed by Valencell, and for the chest strap. In particular, the output waveform shown in FIG. 6B includes a subset of the peaks shown in the waveform of FIG. 6A, which are selected based on respective probabilities of validity thereof (for example, according to the operations described above with reference to FIG. 5B). The waveform of FIG. 6B thus provides a more accurate representation of the RRi time series by including the peaks/sections of FIG. 6A having a higher probability of validity, and excluding the peaks/sections that are more likely attributed to noise.

FIGS. 7A-7B illustrate an example monitoring apparatus 20 configured to be positioned within an ear of a subject according to some embodiments of the present disclosure, although other types of ear worn devices may be utilized. The illustrated apparatus 20 includes an earpiece body or housing 22, a sensor module 24, a stabilizer 25, and a sound port 26. When positioned within the ear of a subject, the sensor module 24 has a region 24a configured to contact a selected area of the ear. The illustrated sensor region 24a is contoured (i.e., is "form-fitted") to matingly engage a portion of the ear between the anti tragus and acoustic meatus, and the stabilizer is configured to engage the anti-helix. However, monitoring devices in accordance with embodiments of the present disclosure can have sensor modules with one or more regions configured to engage various portions of the ear. Various types of devices configured to be worn at or near the ear may be utilized in conjunction with embodiments of the present disclosure.

FIGS. 8A-8B illustrate an example monitoring apparatus 30 including a housing in the form of a sensor band 32 configured to be secured to an appendage (e.g., an arm, wrist, hand, finger, toe, leg, foot, neck, etc.) of a subject. The band 32 includes a sensor module 34 on or extending from the inside surface 32a of the band 32. The sensor module 34 is configured to detect and/or measure physiological information from the subject and includes a sensor region 34a that is contoured to contact the skin of a subject wearing the apparatus 30.

Embodiments of the present disclosure may be utilized in various devices and articles including, but not limited to, patches, clothing, digital cameras (whether wearable, portable, or remote), etc. Embodiments of the present disclosure can be utilized wherever PPG and blood flow signals can be obtained and at any location on the body of a subject. Embodiments of the present disclosure are not limited to the illustrated monitoring devices 20, 30 of FIGS. 7A-7B and 8A-8B. The sensor modules 24, 34 for the illustrated monitoring devices 20, 30 of FIGS. 7A-7B and 8A-8B are configured to detect and/or measure physiological information from a subject wearing the monitoring devices 20, 30. In some embodiments, the sensor modules 24, 34 may be configured to detect and/or measure one or more environmental conditions in a vicinity of the subject wearing the monitoring devices 20, 30.

Embodiments of the present disclosure may utilize a Bayesian framework to determine probabilities for detected peaks in a physiological waveform based on prior/predetermined data indicative of other conditions, in combination with path finding through a graph representing the detected peaks. In particular, in the context of an interval (e.g., n seconds, which may be based on latency requirements) of PPG signal, every possible combination of detected peaks may be analyzed to select a subset indicating the most probable peak locations, thereby excluding peaks of the waveform that are more likely to be attributed to noise. That is, the predetermined data (derived from the physiological waveform itself and/or from outputs of distinct sensors) can be used to effectively remove or filter out portions of a waveform that may be inaccurate with respect to the desired physiological information contained in the waveform. Some embodiments of the present disclosure may thus further extend the Bayesian framework to allow sensor fusion and include additional sensor information in the decision process. Embodiments herein can use a maximum or minimum path finding algorithm to optimize the posterior estimation and improve accuracy, which may provide a flexible tradeoff between latency and accuracy (more latency means more context which helps improve accuracy).

Further embodiments of the present disclosure may provide that the known heart rate value (not RRi values), which is used to generate the probability matrix described in FIG. 3, may be generated by a frequency-domain motion-tolerant method such as that described in U.S. Patent Publication No. 2015/0018636. That is, even if the heart rate value or other predetermined data is derived from the PPG signal, methods used to generate such predetermined data can be distinct and/or orthogonal from that used to generate the RRi, enabling more robustness to identification of motion artifacts when estimating RRi.

Some embodiments of the present disclosure can use known or predetermined information (derived from the PPG signal itself and/or from other sensors that are distinct from the PPG sensor) to improve probability calculation, define a probability matrix including multiple combinations of peaks, which may include non-consecutive peaks, and perform normalization based on amplitudes within respective local intervals around a peak (e.g., a dynamic or varying window, rather than static window including all detected peaks).

The present invention has been described herein with reference to the accompanying figures, in which specific embodiments are shown. This invention may, however, be embodied in many different forms and should not be construed as limited to the embodiments set forth herein. Like numbers refer to like elements throughout. In the figures, certain layers, components or features may be exaggerated for clarity, and broken lines illustrate optional features or operations unless specified otherwise. In addition, the sequence of operations (or steps) is not limited to the order presented in the figures and/or claims unless specifically indicated otherwise. Features described with respect to one figure or embodiment can be associated with another embodiment or figure although not specifically described or shown as such.

It will be understood that when a feature or element is referred to as being "on" another feature or element, it can be directly on the other feature or element or intervening features and/or elements may also be present. In contrast, when a feature or element is referred to as being "directly on" another feature or element, there are no intervening features or elements present. It will also be understood that, when a feature or element is referred to as being "secured", "connected", "attached" or "coupled" to another feature or element, it can be directly secured, directly connected, attached or coupled to the other feature or element or intervening features or elements may be present. In contrast, when a feature or element is referred to as being "directly secured", "directly connected", "directly attached" or "directly coupled" to another feature or element, there are no intervening features or elements present. Although described or shown with respect to one embodiment, the features and elements so described or shown can apply to other embodiments.

The terminology used herein is for the purpose of describing particular embodiments only and is not intended to be limiting of the invention. As used herein, the singular forms "a", "an" and "the" are intended to include the plural forms as well, unless the context clearly indicates otherwise.

As used herein, the terms "comprise", "comprising", "comprises", "include", "including", "includes", "have", "has", "having", or variants thereof are open-ended, and include one or more stated features, integers, elements, steps, components or functions but does not preclude the presence or addition of one or more other features, integers, elements, steps, components, functions or groups thereof. Furthermore, as used herein, the common abbreviation "e.g.", which derives from the Latin phrase "exempli gratia," may be used to introduce or specify a general example or examples of a previously mentioned item, and is not intended to be limiting of such item. The common abbreviation "i.e.", which derives from the Latin phrase "id est," may be used to specify a particular item from a more general recitation.

As used herein, the term "and/or" includes any and all combinations of one or more of the associated listed items and may be abbreviated as "/".

As used herein, phrases such as "between X and Y" and "between about X and Y" should be interpreted to include X and Y. As used herein, phrases such as "between about X and Y" mean "between about X and about Y." As used herein, phrases such as "from about X to Y" mean "from about X to about Y."

Spatially relative terms, such as "under", "below", "lower", "over", "upper" and the like, may be used herein for ease of description to describe one element or feature's relationship to another element(s) or feature(s) as illustrated in the figures. It will be understood that the spatially relative terms are intended to encompass different orientations of the device in use or operation in addition to the orientation depicted in the figures. For example, if a device in the figures is inverted, elements described as "under" or "beneath" other elements or features would then be oriented "over" the other elements or features. Thus, the exemplary term "under" can encompass both an orientation of over and under. The device may be otherwise oriented (rotated 90 degrees or at other orientations) and the spatially relative descriptors used herein interpreted accordingly. Similarly, the terms "upwardly", "downwardly", "vertical", "horizontal" and the like are used herein for the purpose of explanation only unless specifically indicated otherwise.

It will be understood that although the terms first and second are used herein to describe various features or elements, these features or elements should not be limited by these terms. These terms are only used to distinguish one feature or element from another feature or element. Thus, a first feature or element discussed below could be termed a second feature or element, and similarly, a second feature or element discussed below could be termed a first feature or element.

Unless otherwise defined, all terms (including technical and scientific terms) used herein have the same meaning as commonly understood by one of ordinary skill in the art to which this invention belongs. It will be further understood that terms, such as those defined in commonly used dictionaries, should be interpreted as having a meaning that is consistent with their meaning in the context of the specification and relevant art and should not be interpreted in an idealized or overly formal sense unless expressly so defined herein. Well-known functions or constructions may not be described in detail for brevity and/or clarity.

The term "about", as used herein with respect to a value or number, means that the value or number can vary more or less, for example by +/- 20%, +/-10%, +/-5%, +/- 1%, +/- 0.5%, +/-0.1 %, etc.

The terms "sensor", "sensing element", and "sensor module", as used herein, are interchangeable and refer to a sensor element or group of sensor elements that may be utilized to sense information, such as information (e.g., physiological information, body motion, etc.) from the body of a subject and/or environmental information in a vicinity of the subject. A sensor/sensing element/sensor module may comprise one or more of the following: a detector element, an emitter element, a processing element, optics, mechanical support, supporting circuitry, and the like. Both a single sensor element and a collection of sensor elements may be considered a sensor, a sensing element, or a sensor module.

The term "optical emitter", as used herein, may include a single optical emitter and/or a plurality of separate optical emitters that are associated with each other.

The term "optical detector", as used herein, may include a single optical detector and/or a plurality of separate optical detectors that are associated with each other.

The term "wearable sensor module", as used herein, refers to a sensor module configured to be worn on or near the body of a subject.

The terms "monitoring device", "biometric monitoring device" and "biometric monitor", as used herein, are interchangeable and include any type of device, article, or clothing that may be worn by and/or attached to a subject and that includes at least one sensor/sensing element/sensor module. Exemplary monitoring devices may be embodied in an earpiece, a headpiece, a finger clip, a digit (finger or toe) piece, a limb band (such as an arm band or leg band), an ankle band, a wrist band, a nose piece, a sensor patch, eyewear (such as glasses or shades), apparel (such as a shirt, hat, underwear, etc.), a mouthpiece or tooth piece, contact lenses, or the like.

The term "monitoring" refers to the act of measuring, quantifying, qualifying, estimating, sensing, calculating, interpolating, extrapolating, inferring, deducing, or any combination of these actions. More generally, "monitoring" refers to a way of getting information via one or more sensing elements. For example, "blood health monitoring" includes monitoring blood gas levels, blood hydration, and metabolite/electrolyte levels.

The term "headset", as used herein, is intended to include any type of device or earpiece that may be attached to or near the ear (or ears) of a user and may have various configurations, without limitation. Headsets incorporating biometric monitoring devices, as described herein, may include mono headsets (a device having only one earbud, one earpiece, etc.) and stereo headsets (a device having two earbuds, two earpieces, etc.), earbuds, hearing aids, ear jewelry, face masks, headbands, and the like. In some embodiments, the term "headset" may include broadly headset elements that are not located on the head but are associated with the headset. For example, in a "medallion" style wireless headset, where the medallion comprises the wireless electronics and the headphones are plugged into or hard-wired into the medallion, the wearable medallion would be considered part of the headset as a whole. Similarly, in some cases, if a mobile phone or other mobile device is intimately associated with a plugged-in headphone, then the term "headset" may refer to the headphone-mobile device combination. The terms "headset" and "earphone", as used herein, are interchangeable.

The term "physiological" refers to matter or energy of or from the body of a creature (e.g., humans, animals, etc.). In embodiments of the present invention, the term "physiological" is intended to be used broadly, covering both physical and psychological matter and energy of or from the body of a creature.

The term "body" refers to the body of a subject (human or animal) that may wear a monitoring device, according to embodiments of the present invention.

The term "processor" is used broadly to refer to a signal processor or computing system or processing or computing method which may be localized or distributed. For example, a localized signal processor may comprise one or more signal processors or processing methods localized to a general location, such as to a wearable device. Examples of such wearable devices may comprise an earpiece, a headpiece, a finger clip, a digit (finger or toe) piece, a limb band (such as an arm band or leg band), an ankle band, a wrist band, a nose piece, a sensor patch, eyewear (such as glasses or shades), apparel (such as a shirt, hat underwear, etc.), a mouthpiece or tooth piece, contact lenses, or the like. Examples of a distributed processor comprise "the cloud", the internet, a remote database, a remote processor computer, a plurality of remote processors or computers in communication with each other, or the like, or processing methods distributed amongst one or more of these elements. The key difference is that a distributed processor may include delocalized elements, whereas a localized processor may work independently of a distributed processing system. As a specific example, microprocessors, microcontrollers, ASICs (application specific integrated circuit), analog processing circuitry, or digital signal processors are a few non-limiting examples of physical signal processors that may be found in wearable devices.

The term "remote" does not necessarily mean that the "remote device" is a wireless device or that it is a long distance away from a device in communication with a "remote device". Rather, the term "remote" is used to reference a device or system that is distinct from another device or system or that is not substantially reliant on another device or system for core functionality. For example, a computer wired to a wearable device may be considered a remote device, as the two devices are distinct and/or not substantially reliant on each other for core functionally. However, any wireless device (such as a portable device, for example) or system (such as a remote database for example) is considered remote to any other wireless device or system.

Example embodiments are described herein with reference to block diagrams and flowchart illustrations. It is understood that a block of the block diagrams and flowchart illustrations, and combinations of blocks in the block diagrams and flowchart illustrations, can be implemented by computer program instructions that are performed by one or more computer circuits. These computer program instructions may be provided to a processor circuit of a general purpose computer circuit, special purpose computer circuit, and/or other programmable data processing circuit to produce a machine, such that the instructions, which execute via the processor of the computer and/or other programmable data processing apparatus, transform and control transistors, values stored in memory locations, and other hardware components within such circuitry to implement the functions/acts specified in the block diagrams and flowchart block or blocks, and thereby create means (functionality) and/or structure for implementing the functions/acts specified in the block diagrams and flowchart blocks.

These computer program instructions may also be stored in a tangible computer-readable medium that can direct a computer or other programmable data processing apparatus to function in a particular manner, such that the instructions stored in the computer-readable medium produce an article of manufacture including instructions which implement the functions/acts specified in the block diagrams and flowchart blocks.

A tangible, non-transitory computer-readable medium may include an electronic, magnetic, optical, electromagnetic, or semiconductor data storage system, apparatus, or device. More specific examples of the computer-readable medium would include the following: a portable computer diskette, a random access memory (RAM) circuit, a read-only memory (ROM) circuit, an erasable programmable read-only memory (EPROM or Flash memory) circuit, a portable compact disc read-only memory (CD-ROM), and a portable digital video disc read-only memory (DVD/Blu-Ray).

The computer program instructions may also be loaded onto a computer and/or other programmable data processing apparatus to cause a series of operational steps to be performed on the computer and/or other programmable apparatus to produce a computer-implemented process such that the instructions which execute on the computer or other programmable apparatus provide steps for implementing the functions/acts specified in the block diagrams and flowchart blocks. Accordingly, embodiments of the present invention may be embodied in hardware and/or in software (including firmware, resident software, micro-code, etc.) that runs on a processor such as a digital signal processor, which may collectively be referred to as "circuitry," "a module" or variants thereof.

It should also be noted that in some alternative implementations, the functions/acts noted in the blocks may occur out of the order noted in the flowcharts. For example, two blocks shown in succession may in fact be executed substantially concurrently or the blocks may sometimes be executed in the reverse order, depending upon the functionality/acts involved. Moreover, the functionality of a given block of the flowcharts and block diagrams may be separated into multiple blocks and/or the functionality of two or more blocks of the flowcharts and block diagrams may be at least partially integrated. Finally, other blocks may be added/inserted between the blocks that are illustrated. Moreover, although some of the diagrams include arrows on communication paths to show a primary direction of communication, it is to be understood that communication may occur in the opposite direction to the depicted arrows.

Many different embodiments have been disclosed herein, in connection with the above description and the drawings. It will be understood that it would be unduly repetitious and obfuscating to literally describe and illustrate every combination and subcombination of these embodiments. Accordingly, the present specification, including the drawings, shall be construed to constitute a complete written description of all combinations and subcombinations of the embodiments of the present invention described herein, and of the manner and process of making and using them, and shall support claims to any such combination or subcombination.

**Claims**

1. A physiological signal processing method, comprising:
   executing, by at least one processor (40), computer program instructions stored in a non-transitory computer readable medium (60) to perform operations comprising:

   detecting respective peaks in a physiological waveform that represents physiological information collected from a subject over a period of time via at least one wearable device (20; 30) that comprises at least one physiological sensor and is worn by the subject;
   computing probabilities for the respective peaks based on predetermined data indicative of one or more conditions, wherein computing the probabilities includes assigning, for each of the respective peaks, a probability that the respective peak is a valid peak of the collected physiological information;
selecting a subset of the respective peaks based on the probabilities thereof as representing more accurate physiological information for the subject; and
generating an output including the subset of the respective peaks that was selected.

2. The physiological signal processing method of Claim 1, wherein the operations further comprise:

   generating a physiological assessment of the subject based on the output including the subset of the respective peaks that was selected; and/or
   transmitting the output including the subset of the respective peaks that was selected to a remote device.

3. The physiological signal processing method of Claim 2, wherein the more accurate physiological information comprises an R-R time-series including consecutive R-R intervals therein.

4. The physiological signal processing method of Claim 1, wherein selecting the subset of the respective peaks comprises:

   determining combinations comprising sequences of peaks among the respective peaks over the period of time, and
   identifying one of the combinations based on a sum of the probabilities of the sequences of peaks thereof as the subset.

5. The physiological signal processing method of Claim 4, wherein at least some of the sequences of peaks comprise non-consecutive peaks.

6. The physiological signal processing method of Claim 1, wherein the predetermined data is received from one or more sensors that are distinct from the at least one physiological sensor, and optionally wherein the one or more sensors comprise one or more optical sensors and/or motion sensors.

7. The physiological signal processing method of Claim 1, wherein the predetermined data is derived from the physiological waveform.

8. The physiological signal processing method of Claim 1 or 2, wherein the physiological waveform comprises a photoplethysmogram (PPG) signal, and wherein the predetermined data comprises a heart rate value, motion data detected by an accelerometer, and/or energy response signal data.

9. The physiological signal processing method of Claim 8, wherein the more accurate physiological information comprises an R-R time-series including consecutive R-R intervals therein, and the method comprises generating a physiological assessment of the subject based on the output including the subset of the respective peaks that was selected, wherein generating the physiological assessment comprises:

determining whether a heart rate variability metric for the subject is within a predetermined range, wherein the heart rate variability metric is calculated based on a group of the consecutive R-R intervals for the subject, and optionally wherein the data indicative of the predetermined data comprises a heart rate value generated based on frequency domain analysis different from that used to provide the R-R time-series.

10. The physiological signal processing method of Claim 8, wherein the predetermined data comprises the motion data, and wherein computing the probabilities for the respective peaks comprises computing an additional probability matrix where each position in the additional probability matrix contains a probability that a section of the PPG signal comprising the respective peaks is free from motion artifacts based on the motion data.

11. The physiological signal processing method of Claim 10, wherein the one or more conditions comprise an output from an accelerometer or other motion sensor indicating whether the subject is moving and/or how the subject is moving, and wherein computing the probabilities for the respective peaks is based on a correspondence of the respective peaks with an interval corresponding to the motion of the subject, optionally wherein the type of the motion of the subject comprises periodic motion, and wherein ones of the respective peaks that fall within a period of the motion are computed to have a lower probability of validity than ones of the respective peaks that fall outside the period of the motion.

12. The physiological signal processing method of Claim 1, wherein the predetermined data comprises amplitudes of the respective peaks, and wherein one of the respective peaks is computed to have a higher probability of validity based on an amplitude thereof that is comparatively larger among the amplitudes and is the only local maxima, or is computed to have a lower probability of validity based on an amplitude thereof that is comparatively smaller among the amplitudes and is one of a plurality of local maxima.

13. The physiological signal processing method of Claim 1, wherein computing the probabilities comprises:

computing initial probabilities for the respective peaks based on amplitudes thereof; and computing weighted or normalized probabilities for the respective peaks based on the amplitudes thereof relative to adjacent peaks of the respective peaks, and optionally computing the probabilities further comprises:

computing probabilities for respective intervals that include two or more of the respective peaks, wherein the respective intervals occur over the period of time; and determining the probabilities based on the weighted or normalized probabilities for the respective peaks and the probabilities for the respective intervals.

14. The physiological signal processing method of Claim 13, wherein the weighted or normalized probabilities are based on a Gaussian distribution.

15. The physiological signal processing method of Claim 1, wherein the physiological waveform is a time-domain representation or a frequency-domain representation.

16. A physiological signal processing device, comprising:
an electronic circuit comprising a non-transitory computer readable medium having computer program instructions stored therein, and at least one processor that is configured to execute the computer program instructions stored in the non-transitory computer readable medium to perform the method of any of the preceding claims.

17. The physiological signal device of Claim 16, wherein the wearable device comprises an earbud, an audio headset, a wrist strap, a wrist watch, an ankle bracelet, or an armband, and wherein the at least one physiological sensor comprises part of a biometric monitoring device that is integrated within the wearable device.

18. A computer program product for physiological signal processing, the computer program product comprising:
a non-transitory computer readable medium having computer program instructions stored therein that, when executed by at least one processor, causes the at least one processor to perform the method of any of Claims 1 to 15.

**Patentansprüche**

1. Verfahren zur Verarbeitung physiologischer Signale, umfassend:
Ausführen von Computerprogrammbefehlen, die in einem nichtflüchtigen, computerlesbaren Medium (60) gespeichert sind, durch mindestens einen Prozessor (40), um Operationen durchzuführen, die Folgendes umfassen:

Erfassen jeweiliger Spitzen in einer physiologischen Wellenform, die physiologische Informationen darstellt, die über einen Zeitraum von mindestens einer tragbaren Vorrichtung (20; 30) erfasst wurden, das mindestens einen physiologischen Sensor umfasst und von dem Subjekt getragen wird;

Berechnen von Wahrscheinlichkeiten für die jeweiligen Spitzenwerte auf der Grundlage vorbestimmter Daten, die einen oder mehrere Zustände anzeigen, wobei das Berechnen der Wahrscheinlichkeiten das Zuweisen einer Wahrscheinlichkeit für jeden der jeweiligen Spitzenwerte umfasst, dass der jeweilige Spitzenwert ein gültiger Spitzenwert der erfassten physiologischen Informationen ist;

Auswählen einer Teilmenge der jeweiligen Spitzenwerte auf der Grundlage ihrer Wahrscheinlichkeiten als genauere physiologische Informationen für die Testperson; und

Erzeugen einer Ausgabe, die die ausgewählte Teilmenge der jeweiligen Spitzenwerte beinhaltet.

2. Verfahren zur Verarbeitung physiologischer Signale nach Anspruch 1, wobei die Vorgänge ferner umfassen:

Erzeugen einer physiologischen Bewertung der Person auf der Grundlage der Ausgabe, die die ausgewählte Teilmenge der jeweiligen Spitzenwerte beinhaltet; und/oder

Übertragen der Ausgabe, die die ausgewählte Teilmenge der jeweiligen Spitzenwerte beinhaltet, an eine entfernte Vorrichtung.

3. Physiologische Signalverarbeitungsverfahren nach Anspruch 2, wobei die genaueren physiologischen Informationen eine R-R-Zeitreihenfolge umfassen, die aufeinanderfolgende R-R-Intervalle beinhaltet.

4. Verfahren zur Verarbeitung physiologischer Signale nach Anspruch 1, wobei das Auswählen der Teilmenge der jeweiligen Spitzen umfasst:

das Bestimmen von Kombinationen, die Sequenzen von Spitzen unter den jeweiligen Spitzen über den Zeitraum; und

Identifizieren einer der Kombinationen auf der Grundlage einer Summe der Wahrscheinlichkeiten der Sequenzen von Spitzen derselben als Teilmenge.

5. Verfahren zur Verarbeitung physiologischer Signale nach Anspruch 4, wobei mindestens einige der Sequenzen von Peaks nicht aufeinanderfolgende Peaks umfassen.

6. Verfahren zur Verarbeitung physiologischer Signale nach Anspruch 1, wobei die vorbestimmten Daten von einem oder mehreren Sensoren empfangen werden, die sich von dem mindestens einen physiologischen Sensor unterscheiden, und wobei optional der eine oder die mehreren Sensoren einen oder mehrere optische Sensoren und/oder Bewegungssensoren umfassen.

7. Verfahren zur Verarbeitung physiologischer Signale nach Anspruch 1, wobei die vorbestimmten Daten aus der physiologischen Wellenform abgeleitet werden.

8. Verfahren zur Verarbeitung physiologischer Signale nach Anspruch 1 oder 2, wobei die physiologische Wellenform ein Photoplethysmogramm-PPG-Signal umfasst und wobei die vorbestimmten Daten einen Herzfrequenzwert, von einem Beschleunigungsmesser erfasste Bewegungsdaten und/oder Energieresponssignaldaten umfassen.

9. Physiologische Signalverarbeitungsverfahren nach Anspruch 8, wobei die genaueren physiologischen Informationen eine R-R-Zeitreihen mit aufeinanderfolgenden R-R-Intervallen umfassen und das Verfahren das Erzeugen einer physiologischen Bewertung des Probanden auf der Grundlage der Ausgabe umfasst, die die Teilmenge der ausgewählten jeweiligen Spitzenwerte beinhaltet, wobei das Erzeugen der physiologischen Bewertung umfasst:

Bestimmen, ob eine Herzfrequenzvariabilitätsmetrik für die Person innerhalb eines vorbestimmten Bereichs liegt, wobei die Herzfrequenzvariabilitätsmetrik auf der Grundlage einer Gruppe aufeinanderfolgender R-R-Intervalle für die Person berechnet wird, und wobei optional die Daten, die die vorbestimmten Daten anzeigen, einen Herzfrequenzwert umfassen, der auf der Grundlage einer Frequenzbereichsanalyse erzeugt wird, die sich von derjenigen unterscheidet, die zur Bereitstellung der R-R-Zeitreihe verwendet wird.

10. Physiologische Signalverarbeitungsverfahren nach Anspruch 8, wobei die vorbestimmten Daten die Bewegungsdaten umfassen und wobei ein Berechnen der Wahrscheinlichkeiten für die jeweiligen Spitzen das Berechnen einer zusätzlichen Wahrscheinlichkeitsmatrix umfasst, wobei jede Position in der zusätzlichen Wahrscheinlichkeitsmatrix eine Wahrscheinlichkeit enthält, dass ein Abschnitt des PPG-Signals, der die jeweiligen Spitzen umfasst, frei von Bewegungsartefakten auf der Grundlage der Bewegungsdaten ist.

11. Physiologische Signalverarbeitungsverfahren nach Anspruch 10, wobei die eine oder mehreren Bedingungen eine Ausgabe von einem Beschleunigungsmesser oder einem anderen Bewegungssensor umfassen, die angibt, ob sich die Person bewegt und/oder wie sich die Person bewegt, und wobei das Berechnen der Wahrscheinlichkeiten für die jeweiligen Spitzen auf einer Entsprechung der jeweiligen Spitzen mit einem Intervall basiert, das der Bewegung der Person entspricht,
wobei optional die Art der Bewegung der Person eine periodische Bewegung umfasst und wobei diejenigen der jeweiligen Spitzen, die in einen Zeitraum der Bewegung fallen, so berechnet werden, dass sie eine geringere Gültigkeitswahrscheinlichkeit haben als diejenigen der jeweiligen Spitzen, die außerhalb des Zeitraums der Bewegung liegen.

12. Physiologische Signalverarbeitungsverfahren nach Anspruch 1, wobei die vorbestimmten Daten Amplituden der jeweiligen Spitzen umfassen und wobei eine der jeweiligen Spitzen auf der Grundlage einer Amplitude, die unter den Amplituden vergleichsweise größer ist und das einzige lokale Maximum darstellt, mit einer höheren Gültigkeitswahrscheinlichkeit berechnet wird oder auf der Grundlage einer Amplitude, die unter den Amplituden vergleichsweise kleiner ist und eines von mehreren lokalen Maxima darstellt, mit einer geringeren Gültigkeitswahrscheinlichkeit berechnet wird.

13. Physiologische Signalverarbeitungsverfahren nach Anspruch 1, wobei das Berechnen der Wahrscheinlichkeiten umfasst:

Berechnen von Anfangswahrscheinlichkeiten für die jeweiligen Spitzen auf der Grundlage ihrer Amplituden; und
Berechnen gewichteter oder normalisierter Wahrscheinlichkeiten für die jeweiligen Spitzenwerte auf der Grundlage ihrer Amplituden im Verhältnis zu benachbarten Spitzenwerten der jeweiligen Spitzenwerte, und optional umfasst das Berechnen der Wahrscheinlichkeiten ferner:

Berechnen von Wahrscheinlichkeiten für jeweilige Intervalle, die zwei oder mehr der jeweiligen Spitzen umfassen, wobei die jeweiligen Intervalle über den Zeitraum auftreten; und
Bestimmen der Wahrscheinlichkeiten auf der Grundlage der gewichteten oder normalisierten Wahrscheinlichkeiten für die jeweiligen Peaks und der Wahrscheinlichkeiten für die jeweiligen Intervalle.

14. Verfahren zur Verarbeitung physiologischer Signale nach Anspruch 13, wobei die gewichteten oder normalisierten Wahrscheinlichkeiten auf einer Gaußschen Verteilung basieren.

15. Verfahren zur Verarbeitung physiologischer Signale nach Anspruch 1, wobei die physiologische Wellenform eine Zeitbereichsdarstellung oder eine Frequenzbereichsdarstellung ist.

16. Physiologische Signalverarbeitungsvorrichtung, umfassend:
eine elektronische Schaltung, die ein nichtflüchtiges, computerlesbares Medium mit darin gespeicherten Computerprogrammbefehlen und mindestens einen Prozessor umfasst, der so konfiguriert ist, dass er die in dem nichtflüchtigen, computerlesbaren Medium gespeicherten Computerprogrammbefehle ausführt, um das Verfahren nach einem der vorstehenden Ansprüche durchzuführen.

17. Physiologische Signalvorrichtung nach Anspruch 16, wobei die tragbare Vorrichtung einen Ohrstöpsel, einen Audio-Kopfhörer, ein Armband, eine Armbanduhr, ein Fußkettchen oder ein Armband umfasst und wobei der mindestens eine physiologische Sensor einen Teil einer biometrischen Überwachungsvorrichtung umfasst, die in die tragbare Vorrichtung integriert ist.

18. Computerprogrammprodukt zur Verarbeitung physiologischer Signale, wobei das Computerprogrammprodukt umfasst:
ein nicht-transitorisches, computerlesbares Medium mit darin gespeicherten Computerprogrammbefehlen, die, wenn sie von mindestens einem Prozessor ausgeführt werden, den mindestens einen Prozessor veranlassen, das Verfahren nach einem der Ansprüche 1 bis 15 auszuführen.

**Revendications**

1. Un procédé de traitement d'un signal physiologique, comprenant :
l'exécution, par au moins un processeur (40), d'instructions de programme informatique stockées dans un support non transitoire lisible par calculateur (60) pour effectuer des opérations comprenant :

la détection de pics respectifs dans une forme d'onde physiologique qui représente une information physiologique recueillie depuis un patient sur une période de temps via au moins un dispositif à porter sur soi (20 ; 30) qui comprend au moins un capteur physiologique et qui est porté par le patient ;
le calcul de probabilités pour les pics respectifs basées sur des données prédéterminées représentatives d'une ou plusieurs conditions, le calcul des probabilités comprenant, pour chacun des pics respectifs, l'attribution d'une probabilité que le pic respectif soit un pic valide de l'information physiologique recueillie ;

la sélection d'un sous-ensemble des pics respectifs sur la base de leurs probabilités, pour représenter une information physiologique plus précise pour le patient ; et

la génération d'une sortie comprenant le sous-ensemble des pics respectifs qui a été sélectionné.

2.  Le procédé de traitement d'un signal physiologique de la revendication 1, dans lequel les opérations comprennent en outre :

la génération d'une évaluation physiologique du patient sur la base de la sortie incluant le sous-ensemble des pics respectifs qui a été sélectionné ; et/ou

la transmission à un dispositif distant de la sortie incluant le sous-ensemble des pics respectifs qui a été sélectionné.

3.  Le procédé de traitement d'un signal physiologique de la revendication 2, dans lequel l'information physiologique plus précise comprend une série temporelle R-R dans laquelle sont inclus des intervalles R-R consécutifs.

4.  Le procédé de traitement d'un signal physiologique de la revendication 1, dans lequel la sélection du sous-ensemble des pics respectifs comprend :

la détermination de combinaisons comprenant des séquences de pics parmi les pics respectifs sur la période de temps, et

l'identification en tant que ledit sous-ensemble de l'une des combinaisons sur la base d'une somme des probabilités des séquences de leurs pics.

5.  Le procédé de traitement d'un signal physiologique de la revendication 4, dans lequel au moins certaines des séquences de pics comprennent des pics non consécutifs.

6.  Le procédé de traitement d'un signal physiologique de la revendication 1, dans lequel les données prédéterminées sont reçues en provenance d'un ou plusieurs capteurs qui sont distincts de l'au moins un capteur physiologique, et éventuellement dans lequel les un ou plusieurs capteurs comprennent un ou plusieurs capteurs optiques et/ou capteurs de mouvement.

7.  Le procédé de traitement d'un signal physiologique de la revendication 1, dans lequel les données prédéterminées sont dérivées de la forme d'onde physiologique.

8.  Le procédé de traitement d'un signal physiologique de la revendication 1 ou 2, dans lequel la forme d'onde physiologique comprend un signal de photopléthysmogramme (PPG), et dans lequel les données prédéterminées comprennent une valeur de rythme cardiaque, des données de mouvement détectées par un accéléromètre, et/ou des données de signal de réponse énergétique.

9.  Le procédé de traitement d'un signal physiologique de la revendication 8, dans lequel l'information physiologique plus précise comprend une série temporelle R-R dans laquelle sont inclus des intervalles R-R consécutifs, et le procédé comprend la génération d'une évaluation physiologique du patient sur la base de la sortie comprenant le sous-ensemble des pics respectifs qui avait été sélectionné, la génération de l'évaluation physiologique comprenant : la détermination si une métrique de variabilité de rythme cardiaque pour le patient est ou non comprise dans une plage prédéterminée, la métrique de variabilité de rythme cardiaque étant calculée sur la base d'un groupe des intervalles R-R consécutifs pour le patient, ou éventuellement les données représentatives des données prédéterminées comprennent une valeur de rythme cardiaque générée sur la base d'une analyse dans le domaine fréquentiel différente de celle utilisée pour produire les séries temporelles R-R.

10. Le procédé de traitement d'un signal physiologique de la revendication 8, dans lequel les données prédéterminées comprennent les données de mouvement, et dans lequel le calcul des probabilités pour les pics respectifs comprend le calcul, sur la base des données de mouvement, d'une matrice de probabilités additionnelles dans laquelle chaque position de la matrice de probabilités additionnelles contient une probabilité qu'un segment du signal PPG incluant les pics respectifs soit dépourvu d'artefacts de mouvement.

11. Le procédé de traitement d'un signal physiologique de la revendication 10, dans lequel les une ou plusieurs conditions comprennent une sortie provenant d'un accéléromètre ou autre capteur de mouvement indiquant si le patient est ou non en mouvement et/ou la manière dont le patient est en mouvement, et dans lequel le calcul des probabilités pour les pics respectifs est basé sur une correspondance des pics respectifs avec un intervalle correspondant au mouvement du patient,

éventuellement dans lequel le type du mouvement du patient comprend un mouvement périodique, et dans lequel ceux des pics respectifs qui se situent dans une période du mouvement sont calculés pour avoir une probabilité de validité qui soit inférieure à celle des pics respectifs qui se situent hors de la période de mouvement.

12. Le procédé de traitement d'un signal physiologique de la revendication 1, dans lequel les données prédéterminées comprennent les amplitudes des pics respectifs, et dans lequel l'un des pics respectifs est calculé pour avoir une probabilité de validité supérieure sur la base du fait que parmi les amplitudes son amplitude est comparativement plus grande et est le seul maximum local, ou est calculé pour avoir une probabilité de validité inférieure sur la base du fait que parmi les amplitudes son amplitude est comparativement plus faible et est l'un des maxima d'une pluralité de maxima locaux.

13. Le procédé de traitement d'un signal physiologique de la revendication 1, dans lequel le calcul des probabilités comprend :

le calcul de probabilités initiales pour les pics respectifs sur la base de leurs amplitudes ; et le calcul de probabilités pondérées ou normalisées pour les pics respectifs sur la base de leurs amplitudes par rapport à des pics adjacents des pics respectifs, et le calcul des probabilités comprenant éventuellement en outre :

le calcul de probabilités pour des intervalles respectifs qui comprennent deux ou plus des pics respectifs, les intervalles respectifs se situant sur la période de temps ; et la détermination des probabilités sur la base des probabilités pondérées ou normalisées pour les pics respectifs, et des probabilités pour les intervalles respectifs.

14. Le procédé de traitement d'un signal physiologique de la revendication 13, dans lequel les probabilités pondérées ou normalisées sont basées sur une distribution gaussienne.

15. Le procédé de traitement d'un signal physiologique de la revendication 1, dans lequel la forme d'onde physiologique est une représentation dans le domaine temporel ou une représentation dans le domaine fréquentiel.

16. Un dispositif de traitement d'un signal physiologique, comprenant :
un circuit électronique comprenant un support non transitoire lisible par calculateur avec des instructions de programme de calculateur stockées dessus, et au moins un processeur qui est configuré pour exécuter les instructions de programme de calculateur stockées dans le support non transitoire lisible par calculateur pour mettre en œuvre le procédé de l'une des revendications précédentes.

17. Le dispositif de traitement d'un signal physiologique de la revendication 16, dans lequel le dispositif à porter sur soi comprend une oreillette, un casque audio, un bandeau de poignet, une montre bracelet, un bracelet de cheville, ou un brassard, et dans lequel l'au moins un capteur physiologique comprend une partie d'un dispositif de suivi biométrique qui est intégré dans le dispositif à porter sur soi.

18. Un produit de programme informatique pour le traitement d'un signal physiologique, le produit de programme informatique comprenant :
un support non transitoire lisible par calculateur avec des instructions de programme de calculateur stockées dessus qui, lorsqu'elles sont exécutées par au moins un processeur, font en sorte que l'au moins un processeur mette en œuvre le procédé de l'une des revendications 1 à 15.

FIG. 1

FIG. 2

FIG. 3

FIG. 4

FIG. 5A

```
                    ┌──────────────┐
                    │    START     │
                    └──────────────┘
                           │
                           ▼
        ┌────────────────────────────────────┐
        │   DETECT PEAKS IN PHYSIOLOGICAL     │
        │    WAVEFORM COLLECTED FROM          │──── 505
        │   SUBJECT VIA WEARABLE DEVICE       │
        └────────────────────────────────────┘
                           │
                           ▼
        ┌────────────────────────────────────┐
        │    COMPUTE PROBABILITIES            │
        │    FOR THE PEAKS BASED ON           │──── 510
        │    PREDETERMINED DATA               │
        └────────────────────────────────────┘
                           │
                           ▼
        ┌────────────────────────────────────┐
        │  SELECT SUBSET OF THE PEAKS BASED   │
        │   ON THE PROBABILITIES THEREOF      │──── 515
        │  AS REPRESENTING MORE ACCURATE      │
        │    PHYSIOLOGICAL INFORMATION        │
        └────────────────────────────────────┘
                           │
                           ▼
        ┌────────────────────────────────────┐
        │     GENERATE PHYSIOLOGICAL          │
        │     ASSESSMENT BASED ON             │──── 520
        │  THE SELECTED SUBSET OF THE PEAKS   │
        └────────────────────────────────────┘
                           │
                           ▼
                    ┌──────────────┐
                    │     END      │
                    └──────────────┘
```

FIG. 5B

FIG. 6A

FIG. 6B

FIG. 7A

FIG. 7B

FIG. 8A

FIG. 8B

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- US 62611764
- US 8652040 B
- US 8700111 B
- US 8647270 B
- US 8788002 B
- US 8886269 B
- US 8929965 B
- US 2015374240 A, Lee Yong Jin/ Salutron, Inc
- US 2017071487 A, Ritscher
- US 20102865532 A, Farringdon
- US 2009326349 A, McGonigle
- US 9801552 B
- US 8923941 B
- US 8512242 B
- US 20170112447, Aumer
- US 20150018636 A

**Non-patent literature cited in the description**

- **SRINIVAS KUNTAMALLA et al.** An Efficient and Automatic Systolic Peak Detection Algorithm for Photoplethysmographic Signals. *International Journal of Computer Applications*, 01 July 2014, vol. 97 (19), 18-23